# EUROPEAN PATENT APPLICATION

(11) **EP 1 927 663 A2**
(43) Date of publication of application: **04.06.2008**
(21) Application number: 07121088.4
(22) Date of filing: 20.11.2007
(51) Int. Cl.: C12P 7/06, C12M 1/02

(54) **Process and plant for the production of bioethanol and proteins**

(30) Priority: 28.11.2006 IT MI20062281
(71) Applicant: MERLONI PROGETTI S.p.A., 20151 Milano (IT)
(72) Inventor: Merloni, Aristide, 60044, Fabriano (AN) (IT); Marchioni, Marco, 10040, Caprie (TO) (IT); De Togni, Fabrizio, 20052, Monza (IT)
(74) Representative: Dini, Roberto

(57) **Abstract**

The invention relates to a process for the production of bioethanol and high protein content flour by starting from agricultural products such as ryc, maizc, sorghum, wheat and other cereals in general.

To this end, a portion of the mass to be subjected to distillation in order to obtain ethanol as a final product is subjected to proteinous fermentation by using yeasts or fungi.

In accordance with different embodiments of the process, the separation of the fibrous portion of the cereals may take place either upstream or downstream of proteinous fermentation.

The invention also relates to the proteinous flour obtained through the process as well as to the plant implementing said process.

## Description

The present invention relates, according to a general aspect thereof, to the production of the so-called bioethanol, i.e. ethanol or ethyl alcohol obtained through transformation of natural products.

These products in particular comprise, without being limited to, agricultural products having a high carbohydrate and sugar content, such as cereals (e.g. rye, maize, sorghum, wheat, barley, rice, etc.).

The interest in bioethanol is essentially related to its applications as a fuel which can be used in internal combustion engines as an alternative to or in combination with petrol; said interest increases whenever the price of petroleum-based hydrocarbons reaches high levels, as is the case nowadays.

Several industrial methods for the production of bioethanol are known, which are all based on the fermentation of a hydrolyzed mixture of the above-mentioned natural raw materials.

After being suitably cleaned and possibly washed, the latter are milled and mixed with water to form a mixture to which enzymes are then added for the purpose of converting the starch contained in the cereals or in the other products into dextrine and subsequently into glucose. This mixture is then fermented, and ethanol is separated from the obtained mass by distillation; the remaining part is the so-called DDGS (Distilled Dried Grain with Soluble), i.e. a waste product which is used by the animal feedstuff industry.

According to a known alternative method, a separation of the fibrous portion of cereals is also carried out after dry milling and before mixing with water; in this case, ethanol is obtained in the same manner, but the resulting waste material, i.e. dry bran, has a very low commercial value because it is poor in proteins.

In substance, it can be said that the processes known in the art are mainly aimed at maximizing ethanol production, and therefore exploit the raw material in that direction; however, by doing so a poor waste material is obtained, which has very little industrial and commercial value.

The technical problem at the basis of the present invention is therefore to improve this state of things; this means that it proposes a process for the production of bioethanol which comprises implementation steps capable of providing, in addition to ethanol, also a second product having a higher economical value.

The idea for solving this problem is to subject the waste product, obtained from the main fermentation that produces ethanol, to a further proteinous-type fermentation step; this proteinous fermentation can be applied to either only the mass from which the fibrous material obtained by milling has been separated or to the whole milled mass; in this latter case, the separation of the waste fibres takes place downstream of proteinous fermentation.

The features of the process according to the invention are set out specifically in the claims appended hereto; they will become more apparent from the following description concerning a few examples of embodiment of the process as shown in the annexed drawings, wherein:
- Fig. 1 shows a diagram of a plant for the production of bioethanol according to the invention;
- Figs. 2, 3, 4 show diagrams of respective variants of the plant for the production of bioethanol of Fig. 1.

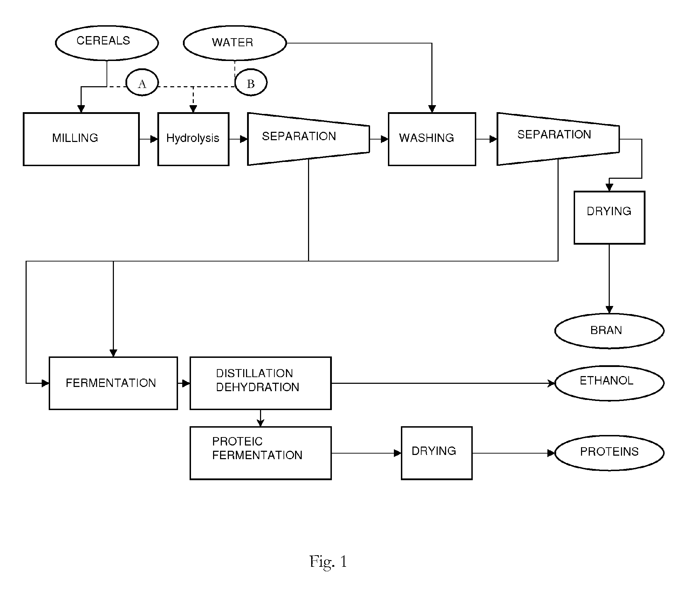

Referring now to Fig. 1, the process illustrated in the diagram produces bioethanol by starting from cereals such as maize, rye, wheat, barley and the like, wherein the separation of the grains from the associated fibres takes place during a milling step.

This step may be carried out either dry or wet, and may possibly be preceded by a washing step (not shown in Fig. 1); in the former case, the cereals are sent directly for milling in a known mill (schematically indicated by a dashed line B in Fig. 1), whereas in the latter case they are diluted with water according to a water/cereal ratio (in weight parts) which may preferably vary from 2 to 4.

Dilution and wet milling are schematically indicated by a dashed line and by reference A in Fig. 1.

Whether the milling is carried out dry or wet, the granulometry of the milled product should preferably be comprised between 400 µm and 1200 µm; in both cases, the obtained mass is subjected to hydrolysis.

The latter occurs by means of enzymes, preferably by executing a number of enzymatic attack cycles which may vary from 1 to 3, depending on the cereal used; thus, for example, three cycles should preferably be used for rye.

Hydrolysis is carried out by using a known reactor; the first cycle, when necessary, is useful for reducing the viscosity of the hydrolyzed mass; the enzymatic mixture/cereal ratio(in weight) is variable from 40 to 250 ppm, while the permanence time is 20-85 minutes; preferably, the reaction occurs at temperatures between 35 °C and 60 °C, with a pH value between 5 and 6.

The second hydrolysis cycle is the transformation of the starch contained in the cereal seed into dextrines; in this case, the weight ratio between enzymatic mixture and cereal may vary between 120 and 300 ppm, for a permanence time which may vary from 60 to 200 minutes at a temperature of 60-90 °C, with pH values within the range of 3.5-6.

The third hydrolysis cycle allows to transform dextrines into glucose; it takes place with an enzymatic mixture/cereal ratio (in weight) which may vary from 300 to 600 ppm, for a permanence time of 240-420 minutes; the temperature during this step is 30-65 °C, while the pH value is 4-5.

The mass thus hydrolyzed is then subjected to separation in a settler, which separates fibre from the liquid portion.

The former then undergoes a washing step, wherein the fibrous fraction is suspended in water again, with a weight ratio of 1-2.5, for the purpose of recovering the glucose which remained imbibed within the fibre; after this washing step, the resulting mass is subjected to a further separation in a second settler, which separates the liquid phase from the fibrous phase.

The former then joins the liquid phase exiting the first settler, while the latter is dried, thus becoming bran.

The liquid phase undergoes a first aerobic alcoholic fermentation process through discontinuous cycles, after filling the fermentation vat (60-90 % of the final volume) and inoculating freeze-dried yeast (e.g. such as Saccharomyces Cerevisiae, Saccharomyces Carlsbergensis, etc.) in quantities which may vary from 1 to 5 % in weight; the duration of this alcoholic fermentation process may vary from 20 to 40 hours at a temperature of 23-36 °C, with a pH value of 3.5-6.

The fermented mass is then distilled and dehydrated through a per se known treatment in order to obtain final ethanol with a purity degree higher than 99.5 %, whereas the residual fraction is subjected to proteinous fermentation.

The latter is an aerobic fermentation process aimed at producing natural proteins through yeasts or fungi isolated in nature, such as: Pichia-Jadinii DSM 70167; Trichoderma-Viride DSM 769; Dekkera-Bruxellensis; Kluyveromyces-Marxianus; Pichia-Stipitis; Brettanomyces-Nardensis; Dekkera-Anomala; Brettanomyces-Claussenii; Pichia-Labacensis; Phlebia-Radiata.

The starting substrate consists of the residual cellulose and hemicellulose part, which has not been attacked by alcoholic fermentation yeasts; this fraction is present within the grounds of the distillation column together with the fermentation yeast, and can be attacked by yeasts/fungi which utilize it for their own growth, thus determining a net production of proteins.

This process takes place in a discontinuous manner after filling the fermentation vat (55-92 % of the final volume) and inoculating a solution of vital yeasts/fungi in a quantity of 1.5 - 6 % v/v of the total volume to be fermented. The process then goes on at a temperature between 23 and 36°C with a pH value between 4 and 6, for an overall duration of the fermentation process between 80 and 160 hours.

The solid substance included in the proteinous fermentation mixture is then dried until a protein concentration between 55 % and 65 % of the final dry weight is obtained.

Compared to the processes known in the art, the above-described ethanol production process offers the advantage that it allows to obtain a second product having a high protein content, which therefore has an interesting economical value; this brings added value to the overall production obtained through the process, which is no longer limited to ethanol but also includes proteins that can be used to advantage in the animal feed industry.

To this end, these proteins are packaged in the form of proteinous flour.

A further advantage provided by said process is the clean flow of substrate (free from inert mass) to be subjected to alcoholic fermentation and consequently to proteinous fermentation, which allows to obtain better fermentation performance (e.g. shorter times) and better hydrodynamic performance (i.e. higher fluidity of the liquid) from the plant.

This implies the possibility of subjecting the whole distillation residue to SCP (Single Cell Protein) fermentation in order to enrich the proteinous content and thus to obtain the above-mentioned second product, which can be packaged as proteinous flour.

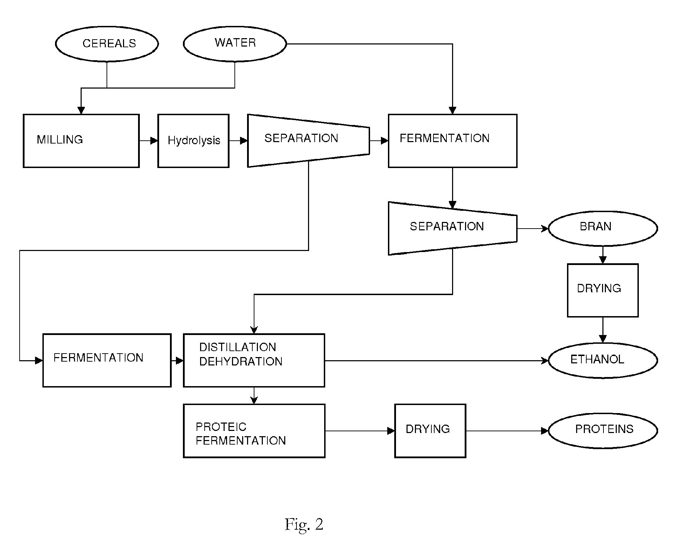

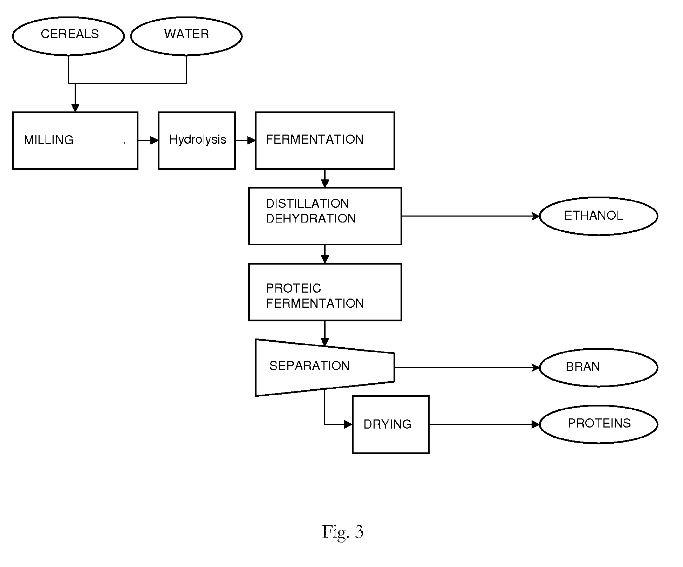

The important results achieved by the above-described process according to the invention can also be obtained by the variants thereof shown in Figs. 2, 3 e4.

Referring to the first variant shown in Fig. 2, it can be said that it differs from the preceding example in that, following the milled cereal hydrolysis step and the subsequent separation step in the settler, the fibrous portion of the hydrolyzed mass is integrated with water and then subjected to alcoholic fermentation.

For this purpose, the fibrous fraction is suspended in water again (ratio from 1 to 3) in order to ferment all the glucose imbibed in the fibre; the alcoholic fermentation process takes place through discontinuous cycles after filling the fermentation vat (60-90 % of the final volume) and inoculating freeze-dried yeast (1-5 % in weight).

The overall duration of the fermentation process may vary between 20 and 40 hours at a temperature of 23 and 36 °C, with a pH value of 3.5-6.

The resulting product is then separated in a settler into a fibrous portion and an alcoholic solution, the former being subsequently dried to produce bran and the second being subsequently subjected to distillation and dehydration together with the liquid exiting the first separator.

According to this variant of the invention, during the bran drying step it is possible to obtain an ethanol fraction which adds to the main volume produced by the process as a result of the above-mentioned distillation and dehydration steps.

In addition to the final ethanol, a residual mixture is also obtained which, as in the preceding case, is then subjected to proteinous fermentation for the purpose of producing natural proteins by means of yeasts or fungi isolated in nature, such as: Pichia-Jadinii DSM 70167; Trichoderma-Viride DSM 769; Dekkera-Bruxellensis; Kluyveromyces-Marxianus; Pichia-Stipitis; Brettanomyces-Nardensis; Dekkera-Anomala; Brettanomyces-Claussenii; Pichia-Labacensis; Phlebia-Radiata).

The starting substrate consists of the residual cellulose and hemicellulose portion, which has not been attacked by the alcoholic fermentation yeasts; this fraction is present within the distillation column dregs together with the fermentation yeast, and can be attacked by yeasts/fungi which utilize it for their own growth, thus determining a net production of proteins.

This process takes place in a discontinuous manner after filling the fermentation vat (55-92 % of the final volume) and inoculating a solution of vital yeasts/fungi (1.5 - 6 % v/v). The process then goes on at a temperature between 23 and 36° C with a pH value between 4 and 6, for an overall duration of the fermentation process between 80 and 160 hours.

The compound derived from proteinous fermentation is then dried, thus obtaining the second product of the process in the form of flour, which is rich in proteins to an extent between 55 and 65 % of the final dry weight.

This first variant of the process offers the advantage of providing increased recovery of the available glucose by carrying out two distinct alcoholic fermentation steps; furthermore, ethanol production is also increased (the conditions being equal to those of Fig. 1), since additional ethanol is obtained during the bran drying step.

A second variant of the process according to the invention is shown in Fig. 3.

As the diagram clearly shows, in this case the separation of the fibrous content of the mixture from which ethanol is obtained takes place only downstream of the proteinous fermentation process used for producing the protein-rich second product.

Therefore, in summary, after the cereals have been milled (either dry or wet as explained), they are diluted with water and subjected to hydrolysis; the hydrolyzed mixture then undergoes alcoholic fermentation through discontinuous cycles, in accordance with the above description in relation to the first example of embodiment of the process.

The same applies to the subsequent distillation and dehydration steps, from which ethanol is obtained; in practice, it can be said that up to this point the process is similar to those currently known in the art.

However, unlike the processes known in the art, in this case the fermented and dehydrated compound (DDGS) obtained on the bottom of the distillation column is sent to proteinous fermentation according to the teaching of the invention.

This fermentation is carried out by using the same yeasts or fungi and the same parameters as those used in the preceding cases; therefore, for simplicity, reference should be made to the above explanations in this regard.

In this second variant, however, the fibrous portion of the fermented compound is separated downstream of proteinous fermentation, thus obtaining bran, while the high protein content portion is dried, thus obtaining the second product of the process according to the invention.

Also this variant of the invention offers the advantage of providing complete ethanol recovery, in that all the milled and hydrolyzed mass is subjected to alcoholic fermentation; furthermore, the centrifugal separations before alcoholic fermentation have been eliminated, thereby avoiding any risk of contamination.

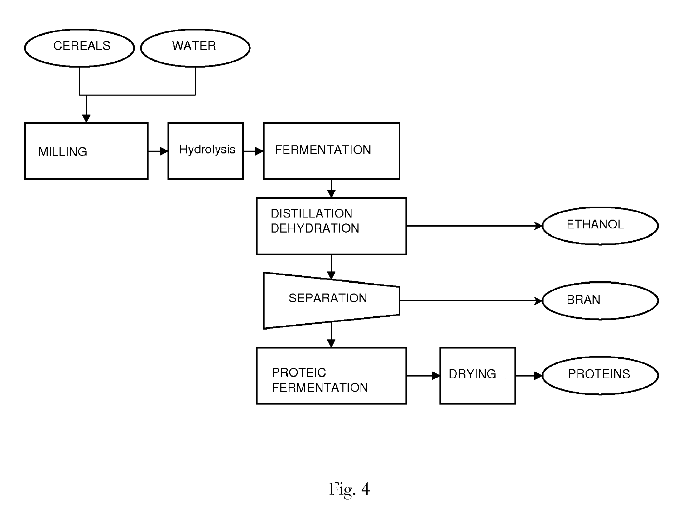

A last variant of the process according to the invention is shown in Fig. 4, where it can be seen that this case differs from the preceding case of Fig. 3 because the separation of bran from the distilled and dehydrated residual compound (DDGS) is carried out before the proteinous fermentation step.

Therefore, this latter step is carried out without the fibrous portion which originates bran, but the resulting product is still the high protein content product described above in relation to the preceding variants of the process according to the invention.

The same considerations pertaining to and advantages offered by the other variants apply to this variant as well, in particular as concerns the production of ethanol through fermentation of the entire cereal mass, i.e. both the fibrous and the useful masses, and the elimination of the centrifugal separation steps which might cause contamination.

All of these variants still fall within the scope of the following claims.

## Claims

**1.** Process for the production of bioethanol, comprising the steps of:
subjecting to hydrolysis a milled agricultural product having a predetermined granulometry;
fermenting and distilling at least a portion of the hydrolyzed product in order to obtain ethanol;
**characterized in that** at least a fraction of the fermented mass used for obtaining ethanol is subjected to a further step of proteinous fermentation, thus obtaining a second product having a high protein content.

**2.** Process according to claim 1, wherein the agricultural product is rich in carbohydrates and/or sugars.

**3.** Process according to claim 1 or 2, wherein the proteinous fermentation is of the aerobic type.

**4.** Process according to claim 3, wherein the proteinous fermentation takes place in a discontinuous manner after filling a fermentation vat and inoculating a solution of vital yeasts and/or fungi.

**5.** Process according to claim 4, wherein the inoculation of yeasts is carried out in quantities within the range of 1.5-6 % (v/v) of the total volume to be fermented.

**6.** Process according to claim 4 or 5, wherein the proteinous fermentation is carried out by using yeasts or fungi among the following: Pichia-Jadinii DSM 70167; Trichoderma-Viride DSM 769; Dekkera-Bruxellensis; Kluyveromyces-Marxianus; Pichia-Stipitis; Brettanomyces-Nardensis; Dekkera-Anomala; Brettanomyces-Claussenii; Pichia-Labacensis; Phlebia-Radiata.

**7.** Process according to any of claims 4 to 6, wherein the proteinous fermentation is carried out at a temperature between 23 and 36° C and with a pH value between 4 and 6.

**8.** Process according to any of the preceding claims, wherein the compound subjected to proteinous fermentation is dried until a protein concentration between 55 % and 65 % of the final dry weight is obtained.

**9.** Process according to any of the preceding claims, wherein the granulometry of the milled product is between 400 µm and 1200 µm.

**10.** Process according to any of the preceding claims, wherein the hydrolysis takes place by means of enzymes.

**11.** Process according to claim 10, wherein the hydrolysis is carried out with a number of cycles of enzymatic attacks which may vary from 1 to 3.

**12.** Process according to claim 11, wherein the first cycle (if necessary) is used for reducing the viscosity of the hydrolyzed mass, and the enzymatic mixture/milled product ratio (in weight) may vary from 40 to 250 ppm.

**13.** Process according to claim 12, wherein the first enzymatic attack cycle takes place at temperatures between 35 °C and 60 °C for a permanence time of 20-85 minutes, with a pH value between 5 and 6.

**14.** Process according to claim 11, wherein the second enzymatic attack cycle is used for transforming the starch contained in the cereal seed into dextrines.

**15.** Process according to claim 14, wherein the weight ratio between enzymatic mixture and cereal may vary from 120 to 300 ppm, for a permanence time which may vary from 60 to 200 minutes at a temperature of 60-90 °C and with pH values within the range of 3.5-6.

**16.** Process according to claim 11, wherein the third enzymatic attack cycle is used for transforming dextrines into glucose.

**17.** Process according to claim 16, wherein the third cycle takes place with an enzymatic mixture/milled product ratio (in weight) which may vary from 300 to 600 ppm, for a permanence time of 240-420 minutes at a temperature of 30-65 °C and with pH values of 4-5.

**18.** Process according to any of the preceding claims, wherein the alcoholic fermentation of the hydrolyzed product takes place through discontinuous cycles, after filling a fermentation vat and inoculating freeze-dried yeast in quantities which may vary from 1 to 5 % in weight.

**19.** Process according to claim 18, wherein the alcoholic fermentation is carried out at temperatures within the range of 23-36 °C and with a pH value of 3.5-6.

**20.** Process according to any of the preceding claims, comprising a fibrous portion separation step carried out downstream of the hydrolysis step, wherein a first fibreless separated fraction is sent to fermentation in order to obtain ethanol, while a second fraction containing the fibres of the agricultural product is subjected to washing with water.

**21.** Process according to claim 20, wherein the fibre of the agricultural product is separated from the fraction subjected to washing with water, and the remaining portion is sent to fermentation.

**21.** Process according to any of claims 1 to 19, comprising a separation step downstream of the hydrolysis step, wherein a first separated fraction is sent to fermentation in order to obtain ethanol, while a second fraction containing the fibres of the product is diluted with water and fermented.

**22.** Process according to claim 21, wherein after fermentation the second fraction is separated from the fibres and the fibreless portion thereof is distilled in order to obtain ethanol.

**23.** Process according to any of claims 1 to 19, wherein the fibres of the agricultural product are separated from the mass subjected to proteinous fermentation.

**24.** Process according to any of claims 1 to 19, wherein the fibres of the agricultural product are separated from the distilled mass in order to obtain ethanol before proteinous fermentation.

**25.** Process according to any of the preceding claims, wherein the agricultural product comprises a cereal.

**26.** Process according to claim 25, wherein the cereal comprises one or more of the following: rye, maize, sorghum, wheat, barley, rice,

**27.** Product having a high protein content, which can be obtained through the process according to any of the preceding claims.

**28.** Product according to claim 27, **characterized by** comprising flour having a protein content between 55% and 65% of the dry weight.

**29.** Plant for implementing the process according to any of claims 1 to 26, **characterized by** comprising a vat for the proteinous fermentation of at least a portion of a mass coming from a distiller.
